(19) Europäisches Patentamt
European Patent Office
Office européen des brevets

(11) **EP 4 067 391 A1**

(12) **EUROPEAN PATENT APPLICATION**

(43) Date of publication:
05.10.2022 Bulletin 2022/40

(21) Application number: 22162229.3

(22) Date of filing: 15.03.2022

(51) International Patent Classification (IPC):
C08F 6/28 (2006.01)        A61L 2/00 (2006.01)
A61M 5/178 (2006.01)       B65B 55/00 (2006.01)
G01N 30/88 (2006.01)       A61M 5/31 (2006.01)
A61M 5/34 (2006.01)

(52) Cooperative Patent Classification (CPC):
C08F 6/28; A61L 2/00; A61M 5/178; A61M 5/3129;
A61M 5/348; B65B 55/00; G01N 30/88

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.03.2021  IT 202100007832

(71) Applicant: Nuova Ompi S.r.l.
35017 Piombino Dese (PD) (IT)

(72) Inventors:
• BESSEGATO, Nicola
I-35017 Piombino Dese, PADOVA (IT)
• CAMPESATO, Lara
I-35017 Piombino Dese, PADOVA (IT)
• ZANETTI, Francesco
I-35017 Piombino Dese, PADOVA (IT)

(74) Representative: Long, Giorgio
Jacobacci & Partners S.p.A.
Piazza Mario Saggin, 2
35131 Padova (IT)

(54) **METHOS FOR EXTRACTING ALDEHYDES FROM THE ADHESIVE OF A SYRINGE**

(57)    The present invention relates to a method for extracting aldehydes from the adhesive used for gluing needles to syringes, and to a method for determining the aldehydes releasable from said syringes, before or after such a treatment.

In particular, the present invention relates to a method for extracting residual aldehydes from syringes comprising a step of treating said syringes at a temperature higher than or equal to 50°C, preferably higher than or equal to 90°C, wherein said treatment step is preferably carried out in an autoclave at a temperature higher than 90°C and at a pressure higher than atmospheric pressure.

FIG.1

EP 4 067 391 A1

**Description**

Technical field of the invention

The present invention relates to a method for extracting aldehydes from the adhesive used for gluing needles to syringes, and to a method for determining the aldehydes releasable from said syringes, before or after such a treatment.

Background art

The development of protein-based medicaments poses major problems of contamination due to the interaction with the primary containers (e.g., syringes) in which they are placed for storage and subsequent administration. Such problems are described for example in paragraphs [0001-0007] of US20160251261A1 and in the article by Denfeng Liu et al., "Interactions between Therapeutic Proteins and Acrylic Acid Leachable," PDA J. Pharm. Sci. And Tech. 2012, 66, 12-19.

A particular class of substances which react negatively with such drugs, and which can be released from the primary container, are aldehydes, in particular formaldehyde and acrolein (but also acetaldehyde and methacrolein), which can be present in the syringe cone due to the adhesive used to attach the needle to the syringe, and released therefrom, in particular due to the interaction with some drugs.

Various methods of decreasing the release of substances from the glues used in syringes are known.

For example, it is known to appropriately vary the emission parameters (wavelength, type of light source, time profile and radiation intensity) used during the glue curing step.

However, such methods tend not to be specific, i.e., they have no particular effect with respect to the reduction of aldehydes, and moreover, when it comes to the reduction of such extractable classes to very low levels, such methods are not sufficiently stable.

It is also known to use autoclave treatments on primary containers, for the purpose of the sterilization thereof. Such a treatment is described in EP3496765A2, for example. However, such a process is generally not suitable for syringes as it can weaken the gluing seal of the needle or damage any rubber components (such as the piston or the needle shield cap) already pre-assembled on the syringe. Accordingly, EtO gas sterilization is preferred for this type of component.

In order to keep the syringe production process under control, it is also important to develop an aldehyde detection method which is capable of determining the presence thereof irrespective of the drug later introduced therein. Such a method must be highly precise and sensitive. It is known to use liquid chromatography as a detection method, but the results obtained depend on many factors, including the substance used for the so-called aldehyde derivatization.

In fact, aldehydes are not detectable per se at low concentrations (ng/mL) if they are not first bound to a substance which allows the discrimination thereof, forming a chromophore product (hydrazone) therewith which is determinable with a simple UV-Vis detector; such a chemical process is referred to as derivatization and allows determining the aldehydes at picomole levels with a significant increase in the detectability limits.

The choice of the derivatization agent also affects the sensitivity of the method; for example, some substances do not allow detecting very small amounts of aldehydes (of the order of ng/ml), because the derivatization yields are insufficient for producing an amount of product the signal of which can be determinable even with the most modern analytical techniques.

An example of an unsuitable binder is pentafluorobenzyl hydroxylamine (PFBHA). Although the reactivity of this molecule with carbonyl groups to generate oximes is known, the development of the reaction is analyte-specific. The determination of an aldehyde reacting with PFBHA is generally possible by a mass spectrometry detector, but the derivatization reaction in this case is very sensitive to the following variables: reaction time, temperature, pH, and reagent amount for each specific aldehyde. The effect of each variable is specific to each aldehyde and makes the overall efficiency of the reaction favorable for some aldehydes but simultaneously unfavorable for others. Therefore, using this derivatization reagent does not allow simultaneously determining the four aldehydes under investigation at ng/mL concentration levels. Furthermore, it has been found that when the tests are performed by filling the syringe with a sample liquid, such as water, the amount of aldehydes detected is also affected by other factors not dependent on the amount and quality of adhesive present in the cone, such as aldehydes present in the plunger or in the water itself.

Based on the above, it is therefore necessary to develop a syringe production method which minimizes the release of aldehydes, without causing a worsening in the release of other substances, and which is independent of the type of drug with which the syringe is filled.

There is also a need to develop an analytical method for the determination of residual aldehydes releasable by a syringe, in particular a syringe subjected to a method for extracting aldehydes from the glue.

Summary of the invention

The first technical problem set forth above is substantially solved by a method for extracting residual aldehydes from syringes comprising the technical features set forth in one or more of the appended claims, the definitions of which form an integral part of the present description for the purpose of sufficiency of description. Therefore, the present invention relates to a method for extracting residual aldehydes from syringes comprising a step of treating said syringes at a temperature higher than or equal to 50°C, preferably higher than or equal to 90°C.

The invention further relates to an analytical method for detecting residual aldehydes releasable from a syringe, the method comprising the following steps:

i) Preparation of the sample to be analyzed by isolating the cone 2 of the syringe 1 comprising the portion of needle 3 inserted therein and the glue 4;

ii) Derivatization treatment of the sample of step i) with a compound of formula Ar-NH-NH$_2$, in which Ar is a phenyl substituted by one or more electron-withdrawing groups;

iii) Analysis of the solution resulting from step ii) by reverse phase HPLC coupled to UV detector.

Further features and advantages of the invention will become apparent from the following description of preferred embodiments, given by way of non-limiting example.

Brief description of the drawings

Figure 1 depicts the cone of a syringe with a coupled needle in section;

Figures 2A, 2B and 2C show graphs showing the reduction of the aldehydes releasable from syringes following the treatment according to the invention.

Detailed description of the invention

In a first aspect, the present invention relates to a method for extracting residual aldehydes from syringes comprising a step of treating said syringes at a temperature higher than or equal to 50°C, preferably higher than or equal to 90°C.

The term "syringe" or "syringes" within the meaning of the present invention means a syringe-needle assembly, in which the needle is coupled to the cone of the syringe by means of an adhesive or glue containing residual aldehydes and in which the syringe does not comprise rubber parts. This glue is typically of the UV-radiation cross-linkable type.

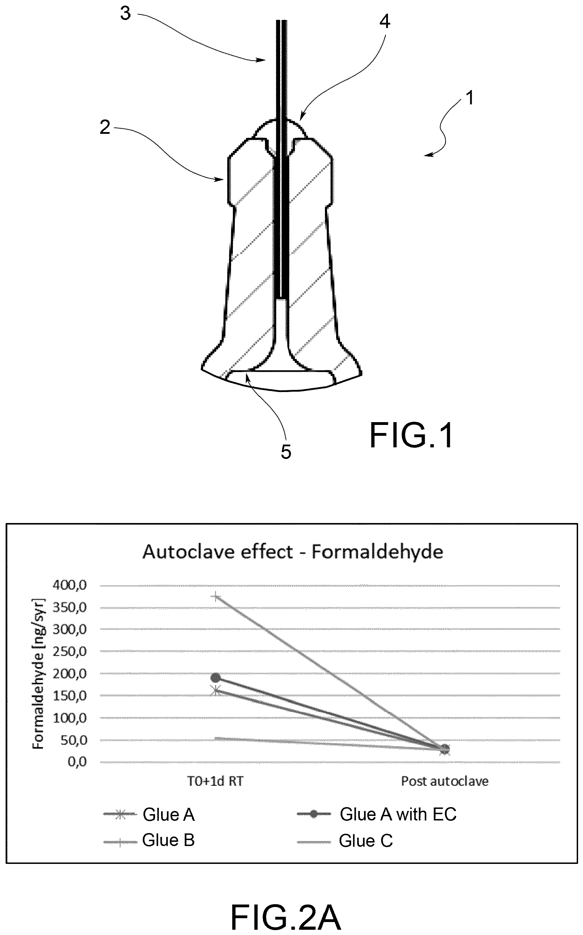

Referring to figure 1, which shows a partial view of a syringe 1, in particular only the section of the cone 2, the conventional method of coupling the needle 3 to the syringe 1 comprises the following steps:

a) preassembling the needle 3 in the cone 2,

b) dosing the glue 4 in the cone 2,

c) drawing the glue 4 from the base of the flange 5,

d) cross-linking the glue with UV lamps (LED or mercury), for example.

In certain embodiments, the pre-assembly step a) is preceded by a plasma treatment under oxygen atmosphere on the needle 3 to be glued. Such a treatment increases the wettability and surface energy of the needle 3, causing a better cleaning of the surface and therefore making it less prone to release contaminants upon gluing, possibly due to the reaction of the material of the needle with the glue.

The method for extracting residual aldehydes from syringes according to the invention comprises, as mentioned, a step of treating said syringes at a temperature higher than or equal to 50°C, preferably higher than or equal to 90°C. Such a treatment step can be carried out under vacuum, at atmospheric pressure in a liquid or gaseous fluid or under vapor pressure. Alternative methods can be:

- Autoclave under water vapor pressure;
- Ultrasonic heated bath;
- Storage at 50°C for 24 hours;
- Vacuum storage at T>50°C with continuous extraction to balance the vacuum, and prevent the vacuum bell from becoming saturated with volatile compounds;
- Washing with water at T=90°C or at boiling point.

A particularly preferred method comprises a step of treating the syringe at a temperature between 110°C and 130°C and at a water vapor pressure between 1.2 and 3 bar. More preferably, the temperature is between 118°C and 123°C and the pressure is between 1.8 and 2.3 bar.

The treatment time is an important parameter, as times of 60 minutes or more, or 90 minutes or more, do not make significant improvements, but tend to worsen the tensile strength of the needle, or even cause it to detach already in the absence of load. The treatment time is thus between 5 and 50 minutes, or between 15 and 40 minutes, or between 15 and 25 minutes, or between 20 and 35 minutes, or between 25 and 30 minutes.

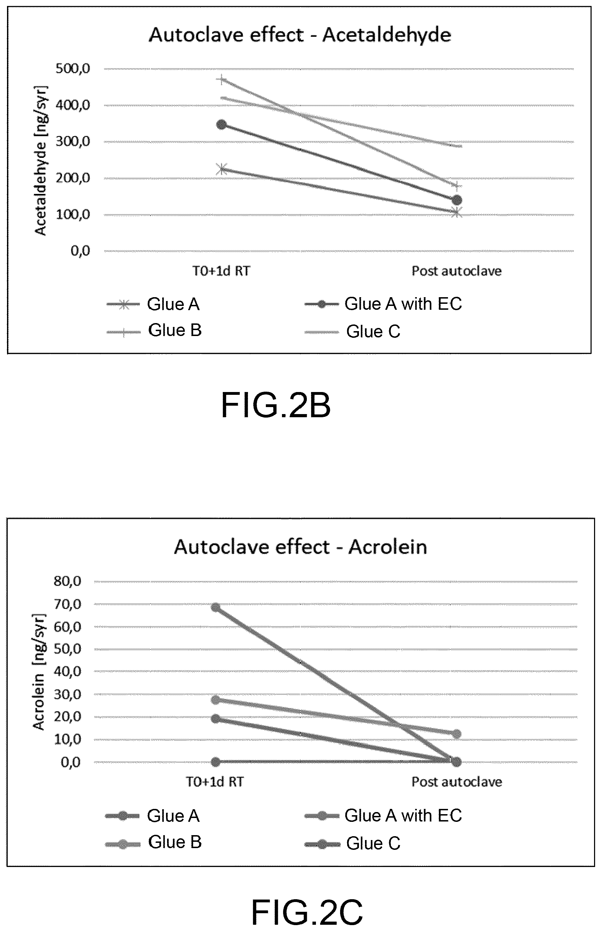

Figures 2A, 2B and 2C depict graphs showing the reduction of formaldehyde, acetaldehyde and acrolein, respectively, released from syringes in which three different glues (glue A, glue B and glue C) with different initial aldehyde contents were used. The glues used are commercial photopolymerized acrylic glues. The measurements were conducted immediately downstream of the syringe production. In all the cases, a substantial reduction in the content of aldehydes releasable from the syringe is achieved, which makes the syringes treated according to the method of the invention usable with any biological fluid, comprising a fluid containing protein material. Glue A was subjected to the autoclaving process with ("glue A with EC" in the graphs) and without *extra-curing,* i.e., with and without a process of further exposure to UV rays with respect to standard times, showing how this parameter does not affect the final result.

Therefore, a syringe obtainable by the method of the invention and having a releasable aldehyde content as follows forms a second object of the invention:

- Formaldehyde < 60 ng/syringe
- Acetaldehyde < 300 ng/syringe, preferably < 200 ng/syringe
- Acrolein < 20 ng/syringe, preferably less than 10 ng/syringe.

The evaluation of the content of aldehydes released from the syringes was carried out by a method developed by the current inventors and forming a further object of the present invention.

Such a method will be described below.

The analytical method of the invention comprises the following steps:

iv) Preparation of the sample to be analyzed by isolating the cone 2 of the syringe 1 comprising the portion of needle 3 inserted therein and the glue 4;

v) Derivatization treatment of the sample of step i) with a compound of formula $Ar-NH-NH_2$, in which Ar is a phenyl substituted by one or more electron-withdrawing groups;

vi) Analysis of the solution resulting from step ii) by reverse phase HPLC coupled to UV detector.

The sample preparation step i) comprises detaching, by means of a shear or other suitable tool, for example, the cone 2 from the cylindrical body of the syringe 1 and removing the protruding part of the needle 3. The portion of syringe 1 thus obtained, consisting of the cone 2, the portion of needle 3 inside the cone 2 and the glue 4 contained therein, further undergoes a crushing treatment of said syringe portion to give said sample.

The sample derivatization treatment step ii) comprises the steps of:

- Suspending said sample in a solution of water/acetonitrile/derivatization solution;
- Heating the suspension thus obtained to a temperature of 40-55°C, preferably about 50°C, and for a time of 1.5-2.5 hours, preferably about 2 hours.

The derivatization treatment allows transforming the aldehydes released by the glues into hydrazones by reaction with the compound of formula $Ar-NH-NH_2$. Such hydrazones are easily detectable by means of UV spectroscopy.

Preferably, said solution of water/acetonitrile/derivatization solution comprises 16-17 parts by volume of water, 1.5-2.5 parts by volume of acetonitrile, and 0.8-1.2 parts by volume of derivatization solution.

The derivatization solution comprises said compound of formula $Ar-NH-NH_2$, where Ar is a phenyl substituted by one or more electron-withdrawing groups, and preferably consists of 120-130 parts by weight of said compound of formula $Ar-NH-NH_2$ in 13-17 parts by volume of acetonitrile, 18-22 parts by volume of a strong acid, preferably 37% hydrochloric acid, and water to a total of 100 parts by volume.

It is important that the pH of the solution is between 1 and 1.2, more preferably about 1.1.

The compound of formula $Ar-NH-NH_2$ is more preferably 2,4-dinitrophenyl hydrazine.

In certain embodiments, it can be necessary or advisable to crystallize 2,4-dinitrophenyl hydrazine prior to use, for example by crystallizing 2,4-dinitrophenyl hydrazine through hot solubilization in acetonitrile and subsequent reprecipitation.

Step iii) of analyzing the solution of a sample resulting from step ii), containing hydrazones of the aldehydes released by the glue in said solution, comprises the steps of:

1) Calibrating an HPLC column, preferably a C18 column, coupled to a UV detector, by means of standard hydrazone calibration samples of said compound of formula Ar-NH-NH$_2$ with formaldehyde, acetaldehyde, acrolein, and methacrolein and using an appropriate eluent;

2) Injecting said sample solution into said calibrated HPLC column;

3) Calculating the area underneath the UV absorption peak of said sample solution;

4) Calculating the residual amount of aldehydes.

The eluent preferably consists of a varying composition mixture of eluent A (H2O/THF 71-74%/26-29% (V/V)) and eluent B (acetonitrile).

Preferably, the operating conditions of the column are as follows:

- Column temperature: 38-42°C, preferably 40°C
- Autosampler temperature: 2-8°C, preferably 5°C
- Injection volume: 100 µl
- UV detector wavelength: 367 nm
- Flow: 1 mL/min
- Eluent A/Eluent B 65/35 to 30/70 to 65/35.

The calculation of the residual amount of aldehydes is carried out by applying the following equation:

$$C_{sample} = (A_{sample} \times C_{standard} \times F \times D \times R)/A_{standard}$$

where

C = concentration
A = area
D = dilution factor = 1
F = conversion factor from derivatized aldehyde to free aldehyde
R = correction factor of the derivatization yield.

Factors R and F are shown in the following table:

| Aldehyde | Factor F | Factor R |
|---|---|---|
| Formaldehyde | 0.1415 | 1.31 |
| Acetaldehyde | 0.1965 | 2.42 |
| Acrolein | 0.2374 | 2.06 |
| Methacrolein | 0.2801 | 1.57 |

It has been shown that the choice of 2,4-dinitro-phenylhydrazine for derivatization leads to very satisfactory results.

The method thus defined has a specificity, that is, an ability to discriminate the substances sought with respect to noise, in accordance with the validation standards required by the main pharmacopoeias.

The quantification limit of the method thus exposed is highly low, and thus adapted to detect even minimal amounts of aldehydes present in the samples analyzed; the lowest quantifiable signal with respect to background noise was around 1-2 ng/ml per syringe.

The coefficient of variation (RSDx100, where RSD= relative standard deviation given by SD/mean) of the measurements according to the method of the invention is less than or equal to 3%.

The method is also stable within 10% with respect to a sample storage time of 2h at room T, or 6h at 5°C.

ALDEHYDE CONTENT REDUCTION TESTS

Table I below summarizes the tests conducted on uncapped syringes using the various aldehyde reduction methods described above. The determination of the amount of residual aldehydes was carried out as described above. In all the cases, Loctite® AA 3345, produced by Henkel, Duesseldorf was used as a glue. The test was carried out on 5 syringes for each type of treatment and the mean value of residual aldehydes $\pm$ S.D. was calculated.

(Standard Deviation).

N.T. treatment: no treatment
Treatment 1: autoclave, P=2 bar, T=121°C, t=20 minutes
Treatment 2: autoclave, P=2 bar, T=121°C, t=60 minutes
Treatment 3: dry vacuum heating, T=60°C, t=60 minutes
Treatment 4: storage at T=50°C, P=atm, t=1 month
Treatment 5: under vacuum at T=ambient, t=24 hours
Treatment 6: ultrasonic bath, P=atm, T=75°C, t=3 hours.

Treatment 1 was also repeated on capped syringes in which a rubber part is present (treatment 7).

Table I

| treatment | Formaldehyde ng/ syringe | Acetaldehyde ng/ syringe | Acrolein ng/ syringe | Methacrolein ng/ syringe |
|---|---|---|---|---|
| N.T. | 37.1 $\pm$ 9.1 | 1266.3 $\pm$ 884.0 | 31.1 $\pm$ 2.4 | 2.7 |
| 1 | 13.8 $\pm$ 2.7 | 236.6 $\pm$ 144.9 | 3.8 $\pm$ 0.0 | 2.9 $\pm$ 03 |
| 2 | 22.1 $\pm$ 13.8 | 395.3 $\pm$ 232.7 | - | 3.0 $\pm$ 0.4 |
| 3 | 145.0 $\pm$ 17.9 | 331.0 $\pm$ 54.0 | 63.5 $\pm$ 5.6 | - |
| 4 | 17.0 $\pm$ 4.8 | 157.8 $\pm$ 87.0 | 21.3 $\pm$ 1.0 | - |
| 5 | 170.7 $\pm$ 35.7 | 263.2 $\pm$ 14.6 | 60.6 $\pm$ 4.2 | - |
| 6 | 22.3 $\pm$ 2.7 | 127.1 $\pm$ 15.7 | 11.8 $\pm$ 0.6 | 2.8 $\pm$ 0.1 |
| 7 | 74.2 $\pm$ 2.7 | 400.2 $\pm$ 46.6 | 7.2 $\pm$ 1.0 | - |

The above data show that the 3-hour ultrasonic bath at 75° and the autoclaving at 121°C, in particular by reducing the time from 60 minutes to 20 minutes, provide the best reduction of aldehyde content.

Treatments 4 and 6, while resulting in a good reduction of formaldehyde and acetaldehyde, do not provide a sufficient reduction of acrolein (particularly critical component) when compared to methods 1 and 2 in an autoclave. Furthermore, they require too long processing times for an industrial implementation thereof.

The information related to the comparison of the data in autoclave at 121°C for 20 minutes with (treatment 1) and without (treatment 7) cap deserve particular discussion. These data indicate that the cap, which has rubber parts, significantly contributes to the release of aldehydes. The sterilization in an autoclave without the presence of rubber parts is thus improved.

It should be noted that the removal of rubber parts to prevent a possible adhesion of the cap to the needle at high temperature due to the softening of the rubber would not be necessary per se, since the materials used for the cap, including the rubber, are made to resist without "stitching" at the sterilization temperatures in the autoclave, a treatment often used by many manufacturers. However, the present inventors have verified that sterilizing in an autoclave with the cap leads to higher aldehyde contamination of the cone, since the material of the cap contains many more aldehydes, and tends to release them even in the cone, as well as in the autoclaving environment (where they are removed).

This results in a syringe which retains, even downstream of the autoclave, a higher content of aldehydes.

By autoclaving the syringe alone, the aldehydes in the syringe are then removed in a more effective manner. On the other hand, the cap, when assembled after autoclaving, does not contribute to the contamination of the syringe by aldehydes, as the contact surface between the cap and the drug is limited to the circular area related to the needle cannula and there is a blast of air between the rubber and the drug.

It is apparent that only some particular embodiments of the present invention have been described, to which those skilled in the art will be able to make all changes required to adapt it to particular applications, without departing from the scope of protection of the present invention.

**Claims**

1. A method for extracting residual aldehydes from syringes, comprising a step of treating said syringes at a temperature higher than or equal to 50°C, preferably higher than or equal to 90°C, wherein said syringe is a syringe (1)-needle (3) assembly, wherein the needle (3) is coupled to the cone (2) of the syringe (1) by means of an adhesive or glue (4) containing residual aldehydes and wherein the syringe (1) does not comprise rubber parts.

2. The method according to claim 1, wherein said treatment step is carried out under vacuum, at atmospheric pressure in a liquid or gaseous fluid or under vapor pressure.

3. The method according to claim 2, wherein said treatment step is carried out according to one of the following methods:

   - Autoclave under water vapor pressure;
   - Ultrasonic heated bath;
   - Storage at 50°C for 24 hours;
   - Vacuum storage at T>50°C with continuous extraction to balance the vacuum, and prevent the vacuum bell from becoming saturated with volatile compounds;
   - Washing with water at T=90°C or at boiling point.

4. The method according to claim 3, wherein said treatment step is carried out at a temperature between 110°C and 130°C and at a water vapor pressure between 1.2 and 3 bar.

5. The method according to claim 4, wherein the temperature is between 118°C and 123°C and the pressure is between 1.8 and 2.3 bar.

6. The method according to claim 4 or 5, wherein the treatment time is between 5 and 50 minutes, or between 15 and 40 minutes, or between 15 and 25 minutes, or between 20 and 35 minutes, or between 25 and 30 minutes.

7. A method of producing a syringe (1) consisting of a syringe (1)-needle (3) assembly, wherein the needle (3) is coupled to the cone (2) of the syringe (1) by means of an adhesive or glue (4) containing residual aldehydes and wherein the syringe (1) does not comprise rubber parts, the cone (2) having a flange (5), such a method comprising the steps of:

   a) preassembling the needle (3) in the cone (2),
   b) dosing the glue (4) in the cone (2),
   c) drawing the glue (4) from the base of the flange (5),
   d) cross-linking the glue with UV LED or mercury lamps, for example,

   and comprising a final treatment step according to any one of claims 1 to 7.

8. The method according to claim 7, wherein the pre-assembly step a) is preceded by a plasma treatment under an oxygen atmosphere on the needle (3) to be glued.

9. A syringe (1) consisting of a syringe (1)-needle (3) assembly, wherein the needle (3) is coupled to the cone (2) of the syringe (1) by means of an adhesive or glue (4) containing residual aldehydes and wherein the syringe (1) does not comprise rubber parts, as obtainable by the method according to any one of claims 1 to 6 or by the method of claim 7 or 8.

10. The syringe (1) according to claim 9, having a releasable aldehyde content as follows:

    - Formaldehyde < 60 ng/syringe
    - Acetaldehyde < 300 ng/syringe, preferably < 200 ng/syringe
    - Acrolein < 20 ng/syringe, preferably < 10 ng/syringe.

**FIG.1**

**FIG.2A**

FIG.2B

FIG.2C

Europäisches
Patentamt

European
Patent Office

Office européen
des brevets

# EUROPEAN SEARCH REPORT

Application Number

EP 22 16 2229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 593 910 A2 (GERRESHEIMER REGENSBURG GMBH [DE]) 15 January 2020 (2020-01-15) * paragraphs [0068] - [0074]; claims 9,10,14 * | 1-10 | INV. C08F6/28 A61L2/00 A61M5/178 |
| X | US 2019/030564 A1 (JOSTEN STEFAN [DE] ET AL) 31 January 2019 (2019-01-31) * paragraph [0079]; claims 7-15 * | 1-10 | B65B55/00 G01N30/88 A61M5/31 A61M5/34 |
| X | WO 2020/118456 A1 (ATS AUTOMATION TOOLING SYSTEMS INC [CA]) 18 June 2020 (2020-06-18) | 9,10 | |
| A | * paragraphs [0002], [0006] - [0028], [0071], [0072]; claims 1-25; figures 8,9 * | 1-8 | |
| X | DE 19 09 833 B1 (OWENS ILLINOIS INC) 26 November 1970 (1970-11-26) | 9,10 | |
| A | * paragraphs [0001], [0007] - [0011], [0020] - [0037]; claims 1-10 * | 1-8 | |

|  |
|---|
| TECHNICAL FIELDS SEARCHED (IPC) |
| C08F G01N A61L B65B A61M |

The present search report has been drawn up for all claims

| Place of search | Date of completion of the search | Examiner |
|---|---|---|
| Munich | 20 July 2022 | Gold, Josef |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 067 391 A1**

## ANNEX TO THE EUROPEAN SEARCH REPORT
## ON EUROPEAN PATENT APPLICATION NO.

EP 22 16 2229

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2022

| Patent document cited in search report | | Publication date | Patent family member(s) | | Publication date |
|---|---|---|---|---|---|
| EP 3593910 | A2 | 15-01-2020 | CN 110694148 | A | 17-01-2020 |
| | | | DE 102018116560 | A1 | 09-01-2020 |
| | | | EP 3593910 | A2 | 15-01-2020 |
| | | | US 2020009329 | A1 | 09-01-2020 |
| US 2019030564 | A1 | 31-01-2019 | CN 109304288 | A | 05-02-2019 |
| | | | DE 102017212974 | A1 | 31-01-2019 |
| | | | EP 3434378 | A2 | 30-01-2019 |
| | | | US 2019030564 | A1 | 31-01-2019 |
| WO 2020118456 | A1 | 18-06-2020 | EP 3894498 | A1 | 20-10-2021 |
| | | | US 2021300821 | A1 | 30-09-2021 |
| | | | WO 2020118456 | A1 | 18-06-2020 |
| DE 1909833 | B1 | 26-11-1970 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160251261 A1

- EP 3496765 A2

**Non-patent literature cited in the description**

- **DENFENG LIU et al.** Interactions between Therapeutic Proteins and Acrylic Acid Leachable. *PDA J. Pharm. Sci. And Tech.,* 2012, vol. 66, 12-19